# EUROPEAN PATENT APPLICATION

(11) **EP 1 658 834 A1**
(43) Date of publication of application: **24.05.2006**
(21) Application number: 05257087.6
(22) Date of filing: 17.11.2005
(51) Int. Cl.: A61F 13/15

(54) **Diaper**

(30) Priority: 17.11.2004 JP 2004332679
(71) Applicant: Koyo Corporation, Yokohama-shi, Kanagawa 247-0014 (JP)
(72) Inventor: Terada, Sadayoshi, 1-chome, Kamakura-shi Kanagawa (JP); Otake, Johshi, 3-chome, Yokosuki-shi Kanagawa (JP)
(74) Representative: Ellis, Katherine Elizabeth Sleigh

(57) **Abstract**

A brief-type diaper that is capable of preventing deformation caused by squeezing of the liquid absorber and capable of preventing leakage around leg openings. The pant-type diaper comprises of a first elastic member (4, 4') arranged adjacent to free ends provided at the periphery of leg openings of the diaper, in which a lengthwise liquid absorber (3) is longitudinally joined inside of the crotch of the brief-type diaper so that transverse pulling power from a second elastic member (5, 5') or elastic belt-like pieces (β) arranged at the exterior sheet (1) is applied to both sides of the liquid absorber (3) extended from the front portion to the back portion.

## Description

The present invention relates to diapers, in particular of the pull up type, which have excellent fitting properties to a human body when worn and when excrement is discharged during wear, this diaper greatly reduces the risk of leakage from the circumference of the crotch.

Diapers, except flat-type diapers, can be classified into tape-type diapers and brief-type diapers both for babies and adults. A tape-type-diaper comprises of a diaper (a liquid absorber) united with a diaper cover, and is used by fastening both front and back portions of a diaper cover with tapes. On the other hand, a brief-type diaper comprises of a diaper (a liquid absorber) set up on a brief, and is worn like underwear.

As we must operate the fastening tape on the occasion of putting on and taking off, a tape-type diaper imposes a burden on a caretaker. In contrast, in recent years, the absence of operating fastening tape heightens one's interest in a brief-type diaper especially as a care article for adults as it does not require the handling of fastening tape.

In general a brief-type diaper is basically comprises of the brief-shaped exterior made of a non-woven fabric and a liquid absorber set up on the inside of the crotch of the brief-shaped exterior. On this kind of brief-type diapers, for the purpose of preventing displacement while being worn, one or a plurality of the elastic member is arranged along a waist opening of the brief-shaped exterior, one or more elastic member is also arranged on a leg opening along its periphery so as to prevent leakage of discharge.

In order to prevent displacement and leakage of excrement to the outside only with the elastic member arranged on the waist and leg openings, it is necessary to ensure that the arranged elastic member has strong contractile power. However, it may excessively put pressure on the stomach or thigh of a brief-type diaper wearer, and cause the wearer discomfort. Therefore, the conventional technology suggests some brief-type diapers that can prevent displacement and leakage of excrement to the outside without causing the wearer discomfort.

In one of the conventional brief-type diapers is a belt-shaped leakage preventing sheet extending toward the waist that is arranged on the inside of a brief-type diaper, placed on the lower side of the waist opening (See Reference 1). The leakage preventing sheet of the brief-type diaper helps the elastic member arranged on the waist opening to prevent displacement and forms a pocket to store excrement in cooperation with the exterior sheet of a brief-type diaper.

In another example of a brief-type diaper, a plurality of the first elastic member is horizontally arranged on the front portion of the brief-type diaper, and a plurality of the second elastic members whose central portion is curved along the crotch are horizontally arranged alike (See Reference 2). On this type of brief-type diaper, it is possible to omit the elastic member that is generally arranged on the waist opening. Another example is the brief-type diaper, wherein standing cuffs extending toward the longitudinal direction of the liquid absorber are arranged on both side edges of the crotch of the liquid absorber, and the elastic member which can longitudinally contract said liquid absorber is arranged under the liquid absorber (See Reference 3).
[Reference 1] Patent application unexamined publication No. 2003-10237
[Reference 2] Patent application unexamined publication No. H10-127687
[Reference 3] Patent application unexamined publication No. 2004-49765

On most of the conventional brief-type diapers, a flat liquid absorber set up on the inside of the crotch is typically adhered to the crotch of the exterior brief with hot melt adhesives. On this kind of brief-type diapers, wearer's exercise, especially walking, often squeeze the liquid absorber adhered to the exterior brief. As a result of compression and squeeze, it is not rare to see it deformed into a braid-shape. This kind of deformation is not preferable because it reduces the absorbency of the liquid absorber.

The object of the present invention is to provide a brief-type diaper that is capable of preventing deformation caused by squeezing of the liquid absorber while being worn, as well as prevention of displacement without increasing the contractile strength of the elastic member provided on the waist opening, and also the purpose of the present invention is to provide a brief-type diaper which has excellent fitting properties to the wearer.

According to a first aspect of the present invention, there is provided a brief-type diaper as specified in claim 1.

According to a second aspect of the present invention, there is provided a brief-type diaper as specified in claim 4.

One example of a brief-type diaper according to the invention comprises of an exterior sheet which is formed by cutting off the center portion of two opposing sides of a rectangular original sheet in such a manner to have a curved opening so that the exterior sheet has a curved edge interposed between two linear edges on either side, and a liquid absorber whose plane form is rectangular-shaped or hourglass-shaped which is adhered longitudinally to the exterior sheet along the center line of said sheet, wherein the exterior sheet is bended double so as to make the liquid absorber inside and superimpose the center line of the sheet, the linear ends facing each other of the exterior sheet are connected to each other, characterized in that:
(a) the ratio of the length of the side that is cut off when curved openings are arranged on the original sheet: 1 (hereinafter referred to as "the maximum length of a curved opening") and the length of the remaining edges that are left without being cut off: 2L is within the a range of 1:80 to 8:9,
(b) the liquid absorber bonded to the exterior sheet is extended toward the direction of the center line of said sheet as striding over the confined area of the exterior sheet formed by two curved openings,
(c) one or a plurality of a first elastic member is arranged along the edges of the curved openings, on the inside of the free ends arranged in the width of 5 to 30 mm from the edges of the curved openings of the exterior sheet, and
(d) one or a plurality of a second elastic member extending toward the crossing direction of the sheet center line in such a manner to connect the portion of the exterior sheet that corresponds to both sides of the liquid absorber extending the confined area of the exterior sheet and the remaining sides of the exterior sheet, the second elastic member is provided by the exterior sheet.

Another example of a brief-type diaper according to the invention comprises of an exterior sheet which is formed by cutting off the center portion of two opposing sides of a rectangular original sheet in such a manner to have a curved opening so that the exterior sheet has a curved edge interposed between two linear edges on either side, and a liquid absorber whose plane form is rectangular-shaped or hourglass-shaped which is adhered longitudinally to the exterior sheet along the center line of said sheet, wherein the exterior sheet is bended double so as to make the liquid absorber inside and superimpose the center line of the sheet, the linear ends facing each other of the exterior sheet are connected to each other, characterized in that:
(a') on said original sheet wherein two curved openings are arranged, the sheet in which three inelastic belt-like pieces (α) and two elastic belt-like pieces (β) are joined on each lengthwise side in the order of (α) (β) (α) (β) (α), said curved openings are provided at the opposing two sides of the original sheet wherein the inelastic belt-like pieces (α) are placed,
(b') the ratio of the length of the side that is cut off when curved openings are arranged on the original sheet: 1 (hereinafter referred to as "the maximum length of a curved opening") and the length of the remaining edges that are left without being cut off: 2L is within the a range of 1:80 to 8:9,
(c') the liquid absorber bonded to the exterior sheet is extended toward the direction of the center line of said sheet as striding over the confined area of the exterior sheet formed by two curved opening,
(d') one or a plurality of a first elastic member is arranged along the edges of the curved openings, on the inside of the free ends arranged in the width of 5 to 30 mm from the edges of the curved openings of the exterior sheet, and
(e') the position of both sides of the liquid absorber extending across the confined area of the exterior sheet are placed piling up and down with each of the two elastic belt-like pieces comprising the exterior sheet.

Embodiments of the present invention are described below, by way of example only, with reference to the accompanying drawings, in which:
FIG 1 is a development showing Example 1 of the brief-type diaper of the present invention; and
FIG 2 is a development showing Example 2 of the brief-type diaper of the present invention.

In the brief-type diaper of the present invention, a liquid absorber provided longitudinally on the crotch is indirectly affected by the shrink or compression of the second elastic members or the elastic belt-like pieces, especially on the front and back portion so as to pull the liquid absorber from side to side constantly, which prevents deformation into a braid which may be caused by squeezing. In addition, the first elastic members contribute to prevent leakage of discharge from the leg opening of the brief-type diaper.

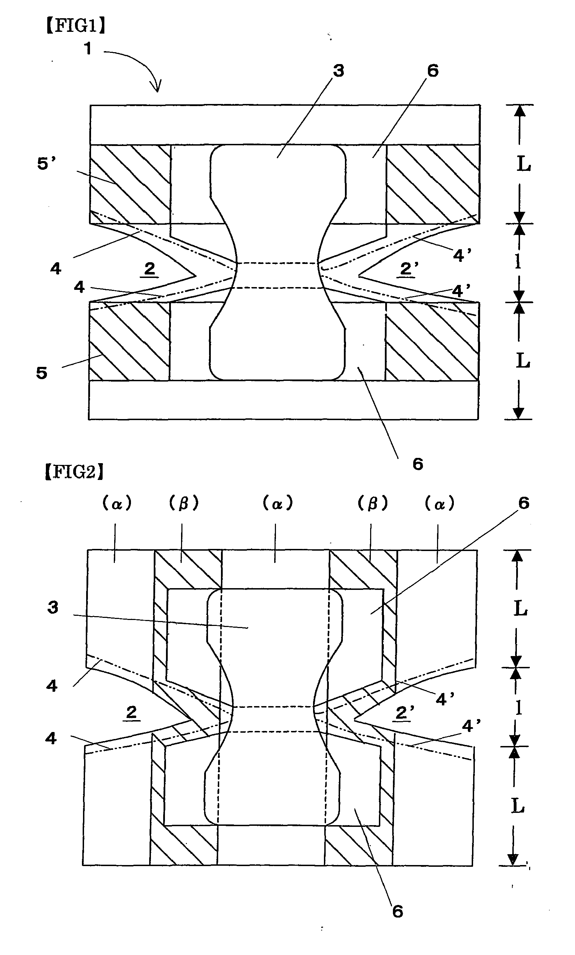

In one example of a brief-type diaper an exterior sheet is prepared from an original sheet which does not have any elasticity itself unless the elastic members are provided. FIG 1 is the development of Example 1, in which the elastic members are not provided.

For an exterior sheet in which the sheet itself does not have elasticity of its own, either hydrophilic or hydrophobic non-woven fabric can be used, and a kind of fiber comprising such a non-woven fabric is welcomed on the present invention. Unless an elastic member is not arranged, the non-woven fabrics which does not express elasticity unless elastic members are provided are called "an inelastic non-woven fabric" in this invention and is distinguished from an elastic non-woven fabric that expresses elasticity on its own.

The form of an original sheet for making an exterior sheet can be square; however, it is generally rectangular.

An exterior sheet 1 shown in FIG 1 comprises two curved openings 2, 2' formed by cutting off the center of the two shorter sides of the rectangular shaped original sheet in a bow shape. The two curved openings 2, 2' are positioned symmetrically to each other relating to the center line of the sheet which is parallel to the above shorter sides.

The ratio of the length 1, which is the length of the side of the original sheet cut off to provide curved openings (hereinafter so called as "curved opening maximum length"), and the length 2L, which is the length of the remaining linear side without being cut off, can be arbitrarily chosen from the range between 1:80 to 8:9. In general, when the ratio of 1:2L becomes smaller, the shape of the diaper will appear more closely to that of trunk. When the ratio of 1: 2L becomes larger, the shape of the diaper will appear more closely to that of a brief. In addition, if two curved openings are formed on the shorter sides of the rectangular shaped original sheet, a brief-type diaper having a relatively large waist can be obtained. If two curved openings are formed on the longer sides, a brief-type diaper having a relatively long space between the crotch and waist.

In the brief-type diaper shown in FIG 1, a liquid absorber 3, of which flat form is hourglass-shaped, is longitudinally arranged along the lengthways of the center line of the exterior sheet 1 in such a manner as adhered to the exterior sheet 1 with hot-melt adhesives or the like. As shown in FIG 1, the rectangular-shaped liquid absorber 3 is provided in such a manner to longitudinally stride over the confined area of the exterior sheet defined by the two left and right curved openings 2, 2'.

In this specification, a confined area of an exterior sheet defined by two curved openings is referred to as "a crotch portion of an exterior sheet", and the other area is referred to as "a front and back portion of an exterior sheet". For a pants-type diaper of the present invention, it is necessary to provide a longitudinal shaped liquid absorber in such a manner to extend from a front to back portion of an exterior sheet.

The breadth (width) of a liquid absorber can be arbitrary chosen from a range without deviating from a confined area of an exterior sheet 1. While a longitudinal length of a liquid absorber can be arbitrarily chosen with a condition that it needs to be longer than the maximum length 1 of two left-right curved openings arranged on an exterior sheet 1 in FIG1; however, generally it is preferable to be set as 1.5 to 3.0 fold longer of the maximum length of the curved openings.

A liquid absorber 3 of the present invention includes mixture of fluffed pulp and a super absorbent polymer, or mixture in which thermally adhesive synthetic fiber is added to the above mixture as a foundation material (core), which is covered with a liquid permeable top sheet and a liquid impermeable back sheet. For a top sheet, hydrophilic non-woven fabric can be employed, and for the back sheet, a liquid impermeable plastic film or a laminated film on which a non-woven fabric is piled up can be employed.' When adhering the liquid absorber 3 to the exterior sheet 1, it is necessary for the back sheet of the liquid absorber to come into contact with the exterior sheet. Normally, hot melt adhesives are used for adhering.

A sheet-type absorber 6 composed of a super absorbent polymer interposed between two non-woven sheets may be used in the brief-type diaper of the present invention. It is preferable for such a sheet-type absorber 6 to have a width larger than that of the liquid absorber 3 as it can prevent leakage around leg openings. The sheet-type absorber 6 is usually provided between the exterior sheet 1 and the liquid absorber 3 by use of hot melt adhesives.

On the curved openings 2, 2' of the exterior sheet 1, free ends are arranged within the range of 5 to 30 mm in width from its periphery, and inside thereof, one or a plurality of elastic members are provided along the periphery of the curved openings. In FIG 1, reference numerals 4, 4' denote parts where a first elastic member is arranged. For an elastic member arranged on the parts, filamentous elastic members or belt-like elastic members made of natural rubber, polyurethane, or segmented polyurethane (Lycra) can be arbitrarily employed. The number of first elastic material to be arranged may be suitably adjusted depending on the contractile power provided around the leg openings of a brief-type diaper.

In a brief-type diaper according to Example 1 shown in FIG 1, in addition to a first elastic member, a second elastic member is provided at the exterior sheet 1. ' Reference numerals 5, 5' in FIG 1 denote the confined area of the second elastic member. As shown in FIG 1, a plurality of the second elastic members are arranged in a direction crossing the center line of the sheet, normally parallel to each other, between the portion of the exterior sheet 1 corresponding to both sides of the liquid absorber 3 extending to the front and back portion of the exterior sheet 1 along the center line of the sheet and the remaining sides of the exterior sheet.

Similar to the first elastic member, filamentous elastic members or the belt-like elastic members made of natural rubber, polyurethane, or segmented polyurethane (Lycra) can be arbitrarily used for the second elastic member.

It is not preferable for the second elastic member arranged on the area 5, 5' of the exterior sheet 1 to extend over the whole area to which the liquid absorber is adhered. However, it is rather preferable for an end of the second elastic member to extend partially to a joined area of the liquid absorber.

The direction of the second elastic member can be either parallel to the top and bottom sides of the exterior sheet 1 in FIG1, or inclined to the top and bottom sides. When inclined, each elastic member preferably inclines downward in such a manner to be symmetrical to the longitudinal center line of the exterior sheet in the area 5 of the front portion, and preferably inclines upward in the area 5' of the back portion. In this case, an inclined angle of the elastic member arranged at both areas can be arbitrarily chosen individually and is preferably at 30 degrees or less.

The number and the interval of the elastic members arranged at the above areas 5 and 5' can be suitably chosen depending on the level of the contractile power required for the said area. In the brief-type diaper of the invention, in addition to the arrangement of the elastic members at the area 5, 5', one or a plurality of elastic members can be arranged around the waist opening when necessity arises.

The exterior sheet 1 of Example 1 does not have elasticity on its own. However, elasticity can be provided to the exterior sheet by arranging the aforementioned first and second elastic members at least at one side of the exterior sheet, and other elastic members depending on necessity. When two and more inelastic non-woven fabrics are used for an exterior sheet material (original sheet), elasticity can be provided to an exterior sheet by laminating a plurality of inelastic non-woven fabrics so as to interpose the first and second elastic members therebetween.

FIG 2 depicts Example 2 of a brief-type diaper of the present invention. This is different from the brief-type diaper of Example 1 shown in FIG 1 at the point that the exterior sheet is prepared from an original sheet that is partially elastic.

That is, an exterior sheet 1 shown in FIG 2 is prepared by arranging three inelastic belt-like pieces (α) and two elastic belt-like pieces (β) in such a manner to be in an order of (α) (β) (α) (β) (α), in which its longer sides come in contact with each other, and join to each longer side adjacent to each other. In this embodiment, an inelastic belt-like piece (α) is positioned at the longer sides of the aforementioned original sheet of a rectangular shape and opposing to each other. The exterior sheet 1 denoted in FIG 2 can be obtained in a same manner as the first example, by cutting off the center of the two longer sides of the original sheet, which oppose to each other, in a bow shape and arranging curved openings 2 and 2' as being symmetrical to the center line of the sheet.

A standard inelastic non-woven fabric can be used for an inelastic belt-like piece (α) comprising an original sheet in FIG 2. For an elastic belt-like piece (β), an elastic non-woven fabric, urethane film, or laminated body of a sandwich structure in which an urethane film interposed between two inelastic non-woven fabrics can be used. Similar to Example 1, an inelastic non-woven fabric and an elastic non-woven fabric can be either be hydrophilic or hydrophobic. For fibers comprising non-woven fabrics, any kind of fibers can be used.

Similar to the example of FIG 1, the ratio of a curved opening's maximum length 1 and a length of a remaining side 2L, which is 1: 2L, is within the range of 1:80 to 8:9. On the curved openings 2, 2' of the exterior sheet 1, free ends are arranged within the range of 5 to 30 mm in width from its periphery, and inside thereof, one or a plurality of elastic members are provided along the periphery of the curved openings. In FIG 2, reference numerals 4, 4' denote parts where a first elastic member is arranged.

In FIG 2, reference numeral 3 denotes a liquid absorber; reference numerals 4, 4' denote the parts where a first elastic member is arranged. Relating to the structure, number, and first elastic members, description for Example 1 can be applied for Example 2 of FIG 2.

Except for the difference of the materials of the exterior sheet, a brief-type diaper of Example 2 is different from the one of Example 1 at the point that a second elastic member is not arranged at the exterior sheet 1, and therefore, the confined areas 5 and 5' of the second elastic member do not exist at the exterior sheet 1 shown in FIG 2.

In Example 2 of FIG 2, two elastic belt-like pieces (β) arranged at the exterior sheet 1 in such a manner to be symmetrical to the longitudinal center line of the exterior sheet to serve as the second elastic member of Example 1. In other words, similar to Example 1, the liquid absorber 3 longitudinally joined along the lengthwise center line of the exterior sheet 1 is extended toward the direction of the center line of the sheet as striding over the confined area of the exterior sheet so as to extend to the front and back portion of the exterior sheet. In the meantime, the liquid absorber 3 is arranged on the exterior sheet 1 in such a manner to stride over the inelastic belt-like pieces located in the lengthwise center of the exterior sheet. Therefore, the tension in the transverse direction can be applied to the liquid absorber 3 shown in FIG 2 due to the effect of the tension in the horizontal direction the two left and right elastic belt-like pieces (β).

## Claims

1. A brief-type diaper comprising an exterior sheet (1), which is formed by cutting off the center portion of two opposing sides of a rectangular original sheet in such a manner to have a curved opening so that the exterior sheet has a curved edge interposed between two linear edges on either side, and a liquid absorber (3) whose plane form is rectangular-shaped or hourglass-shaped which is adhered longitudinally to the exterior sheet along the center line of said sheet, wherein the exterior sheet is bended double so as to make the liquid absorber inside and superimpose the center line of the sheet, the linear ends facing each other of the exterior sheet are connected to each other, **characterized in that**:
(a) the ratio of the length of the side that is cut off when curved openings are arranged on the original sheet: 1 (hereinafter referred to as "the maximum length of a curved opening") and the length of the remaining edges that are left without being cut off: 2L is within the a range of 1:80 to 8:9,
(b) the liquid absorber bonded to the exterior sheet is extended toward the direction of the center line of said sheet as striding over the confined area of the exterior sheet formed by two curved openings,
(c) one or a plurality of a first elastic member (4, 4') is arranged along the edges of the curved openings, on the inside of the free ends arranged in the width of 5 to 30 mm from the edges of the curved openings of the exterior sheet, and
(d) one or a plurality of a second elastic member (5, 5')extending toward the crossing direction of the sheet center line in such a manner to connect the portion of the exterior sheet that corresponds to both sides of the liquid absorber extending the confined area of the exterior sheet and the remaining sides of the exterior sheet, the second elastic member is provided by the exterior sheet.

2. A brief-type diaper as claimed in claim 1, wherein the exterior sheet (1) comprises a sheet of inelastic non-woven fabric, wherein the first (4, 4') or second (5, 5') elastic members are arranged on at least one side of said non-woven fabric.

3. A brief-type diaper as claimed in claim 1, wherein the exterior sheet comprises two sheets of inelastic non-woven fabric, wherein the first (4, 4') or second (5, 5') elastic members are interposed between the two sheets of inelastic non-woven fabric.

4. A brief-type diaper comprises of an exterior sheet (1) which is formed by cutting off the center portion of two opposing sides of a rectangular original sheet in such a manner to have a curved opening so that the exterior sheet has a curved edge interposed between two linear edges on either side, and a liquid absorber (3) whose plane form is rectangular-shaped or hourglass-shaped which is adhered longitudinally to the exterior sheet along the center line of said sheet, wherein the exterior sheet is bended double so as to make the liquid absorber inside and superimpose the center line of the sheet, the linear ends facing each other of the exterior sheet are connected to each other, **characterized in that**:
(a') on said original sheet wherein two curved openings are arranged, the sheet in which three inelastic belt-like pieces (α) and two elastic belt-like pieces (β) are joined on each lengthwise side in the order of (α) (β) (α) (β) (α), said curved openings are provided at the opposing two sides of the original sheet wherein the inelastic belt-like pieces (α) are placed,
(b') the ratio of the length of the side that is cut off when curved openings are arranged on the original sheet: 1 (hereinafter referred to as "the maximum length of a curved opening") and the length of the remaining edges that are left without being cut off: 2L is within the a range of 1:80 to 8:9,
(c') the liquid absorber bonded to the exterior sheet is extended toward the direction of the center line of said sheet as striding over the confined area of the exterior sheet formed by two curved opening,
(d') one or a plurality of a first elastic member (4, 4') is arranged along the edges of the curved openings, on the inside of the free ends arranged in the width of 5 to 30 mm from the edges of the curved openings of the exterior sheet, and
(e') the position of both sides of the liquid absorber extending across the confined area of the exterior sheet are placed piling up and down with each of the two elastic belt-like pieces (β) comprising the exterior sheet.

5. A brief-type diaper as claimed in claim 4, wherein the elastic belt-like piece (β) comprises elastic non-woven fabric or urethane film while the inelastic belt-like piece comprises inelastic non-woven fabric.

6. A brief-type diaper as claimed in claim 4, wherein the elastic belt-like piece (β) comprises a laminated article composed of an urethane film as the middle layer and inelastic non-woven fabric as the outer layers, wherein the inelastic belt-like piece is composed of inelastic non-woven fabric.

7. A brief-type diaper as claimed in claim 2 or 5, between the said absorber and the exterior sheet (1), the sheet-type absorber that can be easily bended is arranged, and the width of the sheet-type absorber is larger than the one of the absorber.
